**Europäisches Patentamt**

**European Patent Office**

**Office européen des brevets**

(19)

(11) Veröffentlichungsnummer: **0 002 213**
**B1**

(12) # EUROPÄISCHE PATENTSCHRIFT

(45) Veröffentlichungstag der Patentschrift:
**04.05.83**

(21) Anmeldenummer: **78101392.5**

(22) Anmeldetag: **18.11.78**

(51) Int. Cl.³: **A 61 N 1/36**, G 08 C 19/22

(54) Sender-Empfänger-System zur Übertragung eines Steuersignals zu einem implantierten Herzschrittmacher.

(30) Priorität: 26.11.77 DE 2753249
27.01.78 DE 2804054

(43) Veröffentlichungstag der Anmeldung:
**13.06.79 Patentblatt 79/12**

(45) Bekanntmachung des Hinweises auf die Patenterteilung:
**04.05.83 Patentblatt 83/18**

(84) Benannte Vertragsstaaten:
**BE CH DE FR GB NL SE**

(56) Entgegenhaltungen:
**DE-A-1 540 676**
**NL-A-7 806 322**
**US-A-3 805 796**
**US-A-4 014 002**
**US-A-4 049 004**

(73) Patentinhaber: **BIOTRONIK Mess- und Therapiegeräte GmbH & Co Ingenieurbüro Berlin, Sieversufer 8, D-1000 Berlin 47 (DE)**

(72) Erfinder: **Blaser, Reinhard, Dr., Bundesratsufer 3, D-1000 Berlin 21 (DE)**
Erfinder: **Teichert, Werner, An der Achternhöfen 19, D-1000 Berlin 47 (DE)**

(74) Vertreter: **Christiansen, Henning, Dipl.-Ing. et al, Unter den Eichen 108a, D-1000 Berlin 45 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Sender-Empfänger-System zur Übertragung eines Steuersignals zu einem implantierten Herzschrittmacher

Die Erfindung betrifft ein Sender-Empfänger-System für implantierte Herzschrittmacher, wie es im Oberbegriff des Patentanspruchs 1 angegeben ist.

Bei implantierten Herzschrittmachern kann es erforderlich werden, eine oder mehrere Betriebseigenschaften des Geräts nach einiger Zeit dem veränderten Krankheitsbild des Trägers anzupassen.

Eine derartige Beeinflussung der Betriebsdaten oder der Betriebsart des Herzschrittmachers sollte im implantierten Zustand vorgenommen werden können, da ein operativer Eingriff stets eine beträchtliche Belastung für den Patienten darstellt.

Die ferngesteuerte Veränderung von Parametern des Schrittmachers wird mittels durch den Körper des Patienten hindurchgelangender Energie vorgenommen.

An ein Sender-Empfänger-System zum Einstellen eines bestimmten Betriebszustandes bei einem implantierten Herzschrittmacher werden eine Reihe von Anforderungen gestellt, die sich von denjenigen bei üblichen technischen Fernsteuerschaltungen unterscheiden. So wird zunächst einmal eine sehr große Betriebssicherheit gefordert, da keine unkontrollierten Veränderungen der eingestellten Schaltzustände durch Fehler in der Schaltung oder durch magnetische Störfelder hervorgerufen werden dürfen. Daneben muß der Stromverbrauch niedrig sein, damit durch die Empfängerschaltung keine wesentliche Vergrößerung der Energiequellen des Schrittmachers bedingt wird. Weiterhin soll die Schaltung auch technisch einfach sein, da zusätzliche Schaltelemente ebenfalls zu einer unerwünschten Vergrößerung des Schrittmachervolumens führen.

Schließlich soll auch die Übertragungsdauer für das Steuersignal klein sein, damit keine unnötigen Wartezeiten für den den Senderteil betätigenden Arzt und den zu betreuenden Patienten entstehen.

Aus der US-PS 39 45 387 ist ein Sender-Empfänger-System der obengenannten Gattung bekannt, bei dem über durch den Körper des Patienten hindurch übertragene Taktimpulse ein im Herzschrittmacher befindlicher Ringzähler weitergeschaltet wird, der nacheinander jeweils ein für einen einzuschaltenden Betriebszustand charakteristisches digital codiertes Steuersignal abgibt, das den gewünschten Zustand auslöst.

Dieses System hat den Nachteil, daß ein bestimmter Betriebszustand nicht direkt angewählt werden kann, so daß in den meisten Fällen ein langwieriges Durchlaufen verschiedener Zählerstände abgewartet werden muß.

Bei einem aus der US-A-4 014 002 bekannten System wird darauf abgestellt, daß Signale beliebiger, d. h. unterschiedlicher Länge übertragen werden sollen. Die Tatsache, daß das beschriebene Signal bei der Übertragung keine separaten Taktsignale aufweisen muß, bedeutet hier, daß in der Empfangseinrichtung auch bei unterschiedliche Anzahlen von zu übertragenden Zeichen aufweisenden Signalzügen die Empfangseinrichtung jeweils durch das Zeichen selbst für den Empfang eines Zeichens (digital kodiertes Signal) vorbereitet wird, so daß hierzu keine separate Taktsteuerung erforderlich ist.

Es ist damit bei dem beschriebenen System wesentlich, daß der Datenempfang sich auf die »Wortlänge« einstellt, d. h. die einzelnen Bits der Datenübertragung für das Empfangssystem so kenntlich sind, daß es auf den Empfang eines Datensignals eingestellt ist. Der Begriff »Taktsignal« dient also in diesem Zusammenhang dazu, das Vorhandensein von Daten zu kennzeichnen, wobei bei dem beschriebenen »selbsttaktenden« Signal hierbei auf zusätzliche Signale verzichtet werden kann, so daß auch bei längeren Datensätzen keine mit den übertragenden Impulsen verbundenen Informationen verlorengehen.

Bei einer weiteren, aus der DE-A-1 540 676 bekannten Einrichtung wird auf das Bedürfnis abgestellt, den Schiebetaktgenerator im Empfänger möglichst einfach ausbilden zu können (Seite 4, Absatz 3, Satz 1). Grundsätzlich geht es dabei um das Ersetzen eines mehrere parallele Übertragungswege erfordernden Fernsteuersystems durch ein einkanalig arbeitendes. Bei der bekannten Einrichtung geht es daher darum, zusammen mit der Möglichkeit, beliebig viele Befehle über einen Kanal zu übertragen, die Erzeugung der Schiebetakte im Fernsteuerempfänger zu vereinfachen. Es geht dabei wiederum vorwiegend um die Frage, wie dem Empfänger gleichzeitig mit dem »Impulsbild, welches die Steuerinformation« enthält, ein das Vorliegen der Information kennzeichnendes Signal (»Schiebetakt«) zu übertragen sei.

Der Erfindung liegt dagegen die Aufgabe zugrunde, ein Sender-Empfänger-System der obengenannten Gattung zu schaffen, das in besonderer Weise an die erwähnten Anforderungen der Herzschrittmachertechnik angepaßt ist, wobei außerdem zu berücksichtigen ist, daß die erzielten Signalübertragungseigenschaften des Systems über einen Zeitraum von mehreren Jahren uneingeschränkt erhalten bleiben müssen.

Es steht damit nicht die Frage im Vordergrund, die übertragenen Informationen als solche für den Empfänger kenntlich zu machen, da die Anzahl der zu empfangenden Informationen festliegt, sondern es besteht die Aufgabe, ein Übertragungsverfahren zu finden, welches sowohl bei absinkender Batteriespannung des Schrittmachers und einer damit veränderten Taktfrequenz des Empfängers als auch bei unterschiedlichen Paarungen von Programmiergeräten und Herzschrittmachern betriebssicher arbeitet.

Diese Aufgabe wird erfindungsgemäß gelöst durch ein System mit den im Kennzeichen des Hauptanspruchs angegebenen Merkmalen.

Die Erfindung beruht auf der Erkenntnis, daß die Anforderungen an die zeitliche Genauigkeit bei der Auswertung der zum Schrittmacher übertragenen Signale wesentlich geringer sind, wenn diese sich jeweils nur auf ein einzelnes Signal (Bit) und nicht auf einen vollständigen Signalzug erstrecken muß, wie es beispielsweise bei der aus der US-A-4 049 004 bekannten Anordnung der Fall ist.

Eine vorteilhafte Ausführung resultiert für binär codierte Steuersignale dann, wenn die binären Einzelsignale einzeln in die das Referenzsignal enthaltenden, zu übertragenden Signalanteile umgesetzt werden. Dadurch, daß die zu übertragenden Impulse durch ihre Flanken bereits selbst ein zeitliches Referenzsignal beinhalten, entfallen zusätzliche Synchronisationsbits, welche zu ihrer Erzeugung, Übertragung und Auswertung erhebliche zusätzliche Mittel notwendig machen würden.

Hervorzuheben sind weiterhin noch diejenigen Maßnahmen, die umfassen, daß die in gewissen Grenzen vorhersehbare Auswirkung der Alterung von zeitbestimmenden Bauelementen im Empfänger nicht durch aufwendige Maßnahmen kompensiert, sondern, wenn die Richtung der Veränderung bekannt ist, in beschränktem Maße gezielt in Kauf genommen wird, wobei der Arzt mit einem Senderteil Betriebsparametereinstellungen an allen hergestellten in Betrieb befindlichen Schrittmachern, die einen Empfänger des erfindungsgemäßen Typs aufweisen, vornehmen kann.

Andererseits kann vorteilhafterweise zusätzlich die Möglichkeit vorgesehen werden, einer ausnahmsweise noch größeren Variation der zeitbestimmenden Bauelemente im Empfänger dadurch zu begegnen, daß auch die Rate der durch den Senderteil abgegebenen Impulse variierbar gemacht wird.

Die Zeitschranken bildenden Signale werden in der Empfängerschaltung in bevorzugter Weise mittels eines Zählers erzeugt, der durch die empfangenen Referenzsignale jeweils auf Null zurückgesetzt wird. Eine solche Zählerschaltung ist hinsichtlich des Stromverbrauchs günstiger als eine sonst üblicherweise als Zeitgeberschaltung verwendete Monoflop-Schaltung. Ein derartiger rücksetzbarer Zähler erweist sich auch deshalb als besonders günstig, weil er, bei geeigneter Auslegung, falls kein RESET-Impuls erscheint, bis zu höheren Zählerständen weiterzählt, bei denen gegebenenfalls zusätzliche Funktionen ausgelöst werden können, wie beispielsweise das Rücksetzen der Logikschaltungen im Empfänger in einen Anfangszustand, falls kein vollständiges, ausführbares Steuersignal empfangen wurde.

Die Störsicherheit der Steuersignalübertragung wird weiter dadurch verbessert, daß zusätzliche redundante Signale vorgesehen sind, die sich von den Signalen, bei fehlerhafter Signalübertragung unterscheiden, wobei es eine besonders günstige Lösung darstellt, wenn diese redundanten Signalanteile die relevanten Signalteile zeitlich umrahmen. Fallen infolge von Störungen Signalteile aus oder werden zusätzliche Impulse eingeschoben, so verschieben sich die redundanten Signalanteile innerhalb der übertragenen Zeichenfolge. Durch eine geeignete Wahl der redundanten Signalanteile kann es erreicht werden, daß, wenn eine derartige Verschiebung im empfangenen Signal auftritt, mit großer Wahrscheinlichkeit nicht eine solche Signalkonfiguration erscheint, die vom Empfänger als Steuersignal akzeptiert wird. Die zeitbestimmenden Eigenschaften der Empfängerschaltung betreffende Änderungen können dabei nicht nur durch altersbedingte Veränderungen der Bauelemente, sondern auch durch ein Nachlassen der Batteriespannung infolge zunehmender Erschöpfung der Energiequellen hervorgerufen werden.

Weiterhin besteht bei programmierbaren Schrittmachern der Wunsch, zusätzlich kurzfristig eine charakteristische Impulsfrequenz einzustellen, um durch Ausmessung der sich tatsächlich einstellenden Anregungsfrequenz Aufschluß über den Zustand der Batterie und damit auch die voraussichtliche restliche Betriebszeit des Schrittmachers zu gewinnen.

Es ist bei nicht programmierbaren Schrittmachern bekannt, den Batteriezustand nach der Implantation dadurch festzustellen, daß ein Dauermagnet in der Nähe des Schrittmachers gebracht wird. Das Magnetfeld des Dauermagneten beeinflußt einen in dem Schrittmachergehäuse eingebauten Reed-Schalter, welcher seinerseits die Anregungsrate des Schrittmachers über entsprechende elektrische Schaltungsmittel auf einen festen Wert einstellt, der jedoch von der Batteriespannung abhängig ist. Durch Auszählen der sich auf diese Weise einstellenden Betriebsfrequenz des Schrittmachers ist es — wie erwähnt — möglich, anhand eines die Spannungs-Frequenzcharakteristik wiedergebenden Diagramms den Zustand der Batterie zu bestimmen.

Bei programmierbaren Schrittmachern bisheriger Bauart ist eine einfache Möglichkeit der Batterieüberprüfung anhand einer einzigen Spannungs-Frequenz-Kennlinie in dieser direkten Weise nicht gegeben, weil die Schrittmacherfrequenz auf verschiedene Werte eingestellt werden kann. Zwar wäre es denkbar, für jede einzelne der programmierbaren Frequenzen eine besondere Spannungs-Frequenz-Kennlinie anzugeben. Hierdurch könnten aber einerseits Irrtümer hervorgerufen werden und zum anderen ergibt sich eine zusätzliche Schwierigkeit daraus, daß die aus dem zunehmenden Abfall der Batteriespannung im Verlaufe der Batterielebensdauer resultierende Frequenzänderung im Extremfall fünf bis sechs Impulse betragen kann. Damit ist die Aussage anhand einer solchen Kennlinie nicht eindeutig, vor allem, wenn die durch Programmierung einstellbaren Frequenzwerte einander dicht benachbart sind.

Es besteht demnach gemäß einer Weiterbildung der Erfindung die Aufgabe, eine Schaltung anzugeben, die auch ohne Benutzung des zur Programmierung erforderlichen Senderteils bei programmierbaren künstlichen Herzschrittmachern erlaubt, eine charakteristische Anregungsfrequenz über ein Dauerstrichsignal von derjenigen Art, wie es zum Auswählen der Betriebszustände verwendet wird, vorübergehend einzustellen und unter Vermeidung der vorgenannten Nachteile ohne größeren zusätzlichen Aufwand einem derartigen Schrittmacher hinzugefügt werden kann, wobei sich auch keine nennenswerte Vergrößerung der Bauform ergeben soll.

Diese Aufgabe wird erfindungsgemäß gelöst durch ein System mit den im Kennzeichen des Unteranspruchs 17 angegebenen Merkmalen.

Die das kurzfristige Umschalten der Anregungsrate auf eine charakteristische Frequenz betreffende Lösung entsprechend der Weiterbildung der Erfindung weist den Vorteil auf, daß der Schrittmacher nicht während der Überprüfung bezüglich seiner eingespeicherten, programmierten Frequenz verändert wird. Dadurch sind Irrtümer und Fehler bei der Rückkehr in den permanenten Betriebszustand ausgeschlossen.

Auch kann, wenn bei Programmiermöglichkeit über ein Magnetfeld der Schrittmacherträger sich unbeabsichtigt in den Bereich eines starken Dauermagnetfeldes begibt, keine Gefahr für diesen eintreten, da sich der Schrittmacher höchstens für den Zeitraum des Verbleibens im Magnetfeld auf die Prüffrequenz umstellt, welche im Rahmen der üblichen Anregungsraten liegt und bei ihrem Auftreten ein Signal für den Schrittmacherträger sein kann, den Bereich des starken Magnetfelds zu verlassen.

Andere vorteilhafte Weiterbildungen der Erfindung sind in den übrigen Unteransprüchen angegeben. Ein bevorzugtes Ausführungsbeispiel einschließlich zweier Ausführungsvarianten ist in den Zeichnungen dargestellt und wird nachfolgend näher beschrieben. Es zeigen:

Fig. 1 eine Darstellung des prinzipiellen Impulsverlaufes des zu übertragenden Signals bei dem beschriebenen Ausführungsbeispiel der Erfindung,

Fig. 2 ein Blockschaltbild des Ausführungsbeispiels,

Fig. 3 eine detaillierte Schaltung des Senders des Ausführungsbeispiels des erfindungsgemäßen Systems,

Fig. 4 eine detaillierte Schaltung des Empfängers des selben Systems, bei dem die erste Ausführungsform einer Weiterbildung der Erfindung realisiert ist,

Fig. 4a eine Variante eines Teils der Schaltung gemäß Fig. 4, wobei dieser Schaltungsteil mit der übrigen Schaltung nach Fig. 4 eine Schaltung entsprechend der zweiten Ausführungsform einer Weiterbildung der Erfindung bildet,

Fig. 5 eine Darstellung des impulsförmigen Spannungsverlaufs an verschiedenen Punkten der in den Fig. 3, 4 und 4a dargestellten Schaltungen,

Fig. 6 ein Blockschaltbild der ersten Ausführungsform der Weiterbildung der Erfindung und

Fig. 6a ein Blockschaltbild der zweiten Ausführungsform der Weiterbildung der Erfindung.

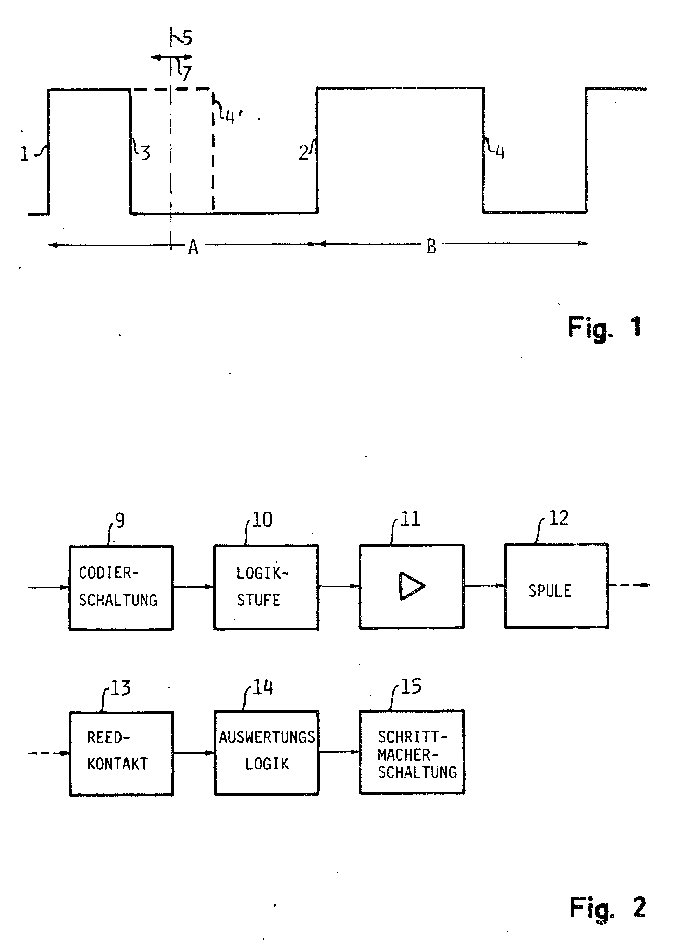

In Fig. 1 ist der prinzipielle Verlauf der Impulse des vom Senderteil seriell auszusendenden Signals dargestellt. Es handelt sich dabei um Impulse, die bei einer mit besonders geringem konstruktiven Aufwand ausführbaren Systemkonfiguration erzeugt werden können, die zur Erleichterung des Verständnisses in einem idealisierten Verlauf der nachfolgenden Beschreibung zugrundegelegt werden soll. Dem dargestellten Signalverlauf liegt ein binär codiertes Steuersignal zugrunde, das insgesamt 8 Bits aufweist und in Fig. 5 oben (2. Zeile) geschlossen dargestellt ist. Drei für das Signal relevante Bits sind mit »X« bezeichnet. Da das zugrundeliegende Steuersignal binär codiert ist, kann das weitere Signal in Bezug auf das Referenzsignal zwei verschiedene zeitliche Lagen einnehmen. Es ist ersichtlich, daß bei entsprechend komplizierten Systemen komplexere Informationsanteile durch eine feinere zeitliche Stufung übertragen werden können.

Bei dem in Fig. 1 dargestellten Signalverlauf, der einen Ausschnitt aus einem seriell zu übertragenden Signal darstellt, bilden die Vorderflanken 1 und 2 der Impulse die Referenzsignale, während die weiteren Signale, deren zeitliche Lage von der Empfängerschaltung ausgewertet wird, durch die Rückflanken der Signale 3 und 4 gebildet werden. Der durchgezogen dargestellte Signalverlauf im Bereich A wird durch ein binäres »1«-Signal erzeugt, während zu dem Signalverlauf im Bereich B das binäre Signal »0« gehört. Bei der Signalübertragung braucht der Abstand der die Referenzsignale bildenden Vorderflanken 1, 2 usw. der Impulse untereinander nicht konstant zu sein. Will man eine zeitliche Kompression der Übertragung erreichen, so kann die Vorderflanke 2 auch nahezu unmittelbar auf die Rückflanke 3 folgen, was allerdings mit dem Nachteil behaftet ist, daß die Ausgabe der Impulse senderseitig nicht nach einem festen Taktschema erfolgen kann. Im Bereich A der Darstellung in Fig. 1 ist bei 4' zusätzlich der Signalverlauf für ein »0«-Signal dargestellt. Als strichpunktierte Linie ist eine Zeitschranke 5 wiedergegeben, die empfängerseitig zur Entschlüsselung des empfangenden Steuersignals gebildet wird. Diese Zeitschranke wird — in Form von entsprechenden logischen Signalen — im Empfänger auf das Erscheinen des zeitlichen Referenzsignals (hier der Vorderflanken 1 oder 2) hin in einem festen zeitlichen Abstand erzeugt.

Dadurch, daß bei dem System gemäß der Erfindung die Lage der Zeitschranken jeweils auf ein Referenzsignal, wie hier die Vorderflanken 1, 2 der Impulse bezogen ist, werden die Anforderungen an die Konstanz der zeitbestimmenden Glieder in der Empfängerschaltung verringert. Beim dargestellten Beispiel, wenn acht Impulse Logikschaltungen 25, 28 und 29 insgesamt mit logischer »Oder«-Verknüpfung an den Eingang des Verstärkers 11, so daß sich insgesamt für den ausgesendeten Signalverlauf das in der neunten Zeile von Fig. 5 dargestellten Bild ergibt.

Nach acht Impulsen des Flip-Flops 23 erreicht der durch positive Flanken getriggerte abwärtszählende Zähler 19 sein Minimum und über den Ausgang »CARRY OUT« gelangt ein L-Impuls an das NAND-Gatter 18, so daß der Oszillator (NAND-Gatter 20 und 21) gestoppt wird. Ein mittels des NAND-Gatters 30 und der durch den Widerstand R 7 und den Kondensator C 5 gebildeten Impulsformstufe erzeugter Impuls setzt die beiden Flip-Flops 22 und 23 in ihre Ausgangslage zurück.

An einem weiteren Eingang des NAND-Gatters 30 ist eine Schaltung angeschlossen, die nach dem Einschalten der Versorgungsspannung die Flip-Flops 22 und 23 ebenfalls in ihre Ausgangslage zurücksetzt (Ausgänge auf L-Pegel).

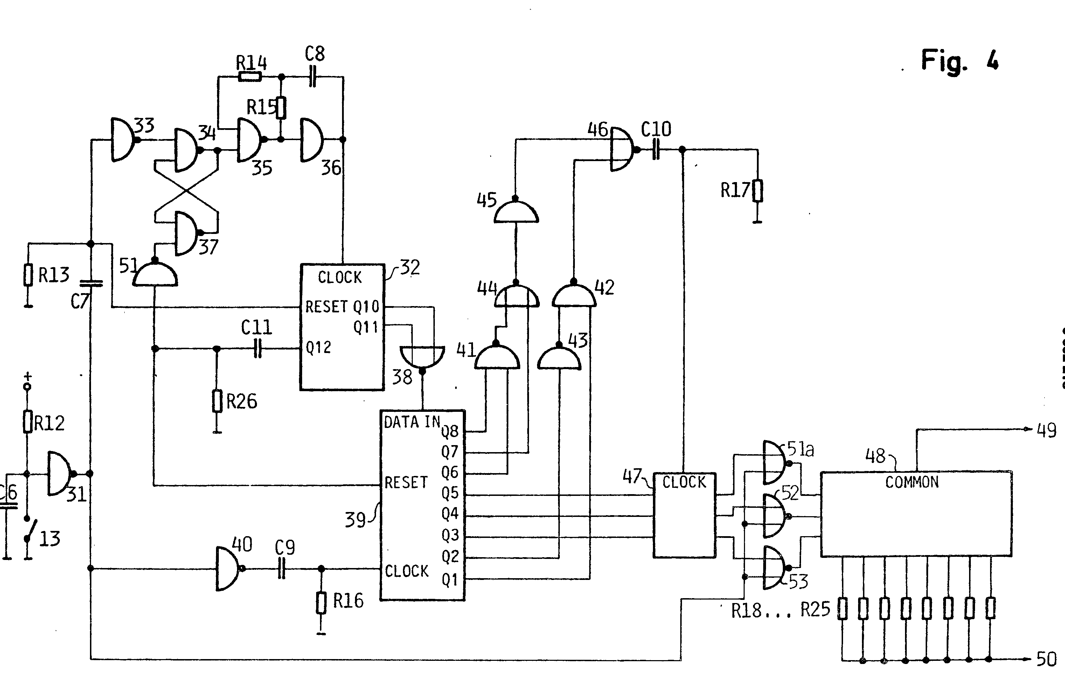

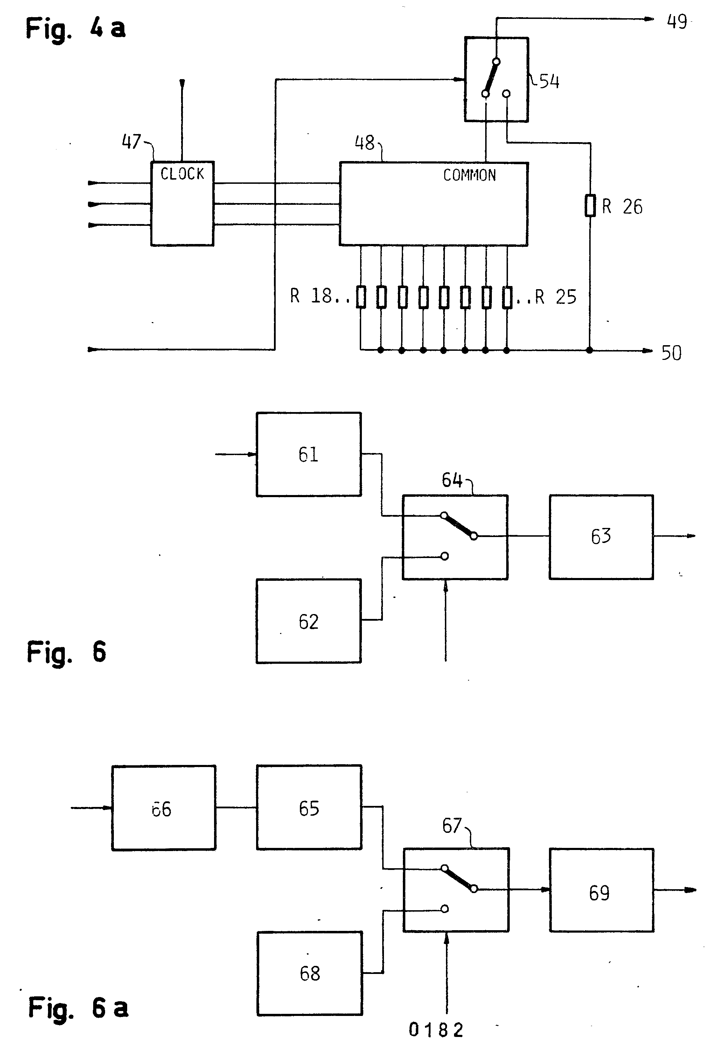

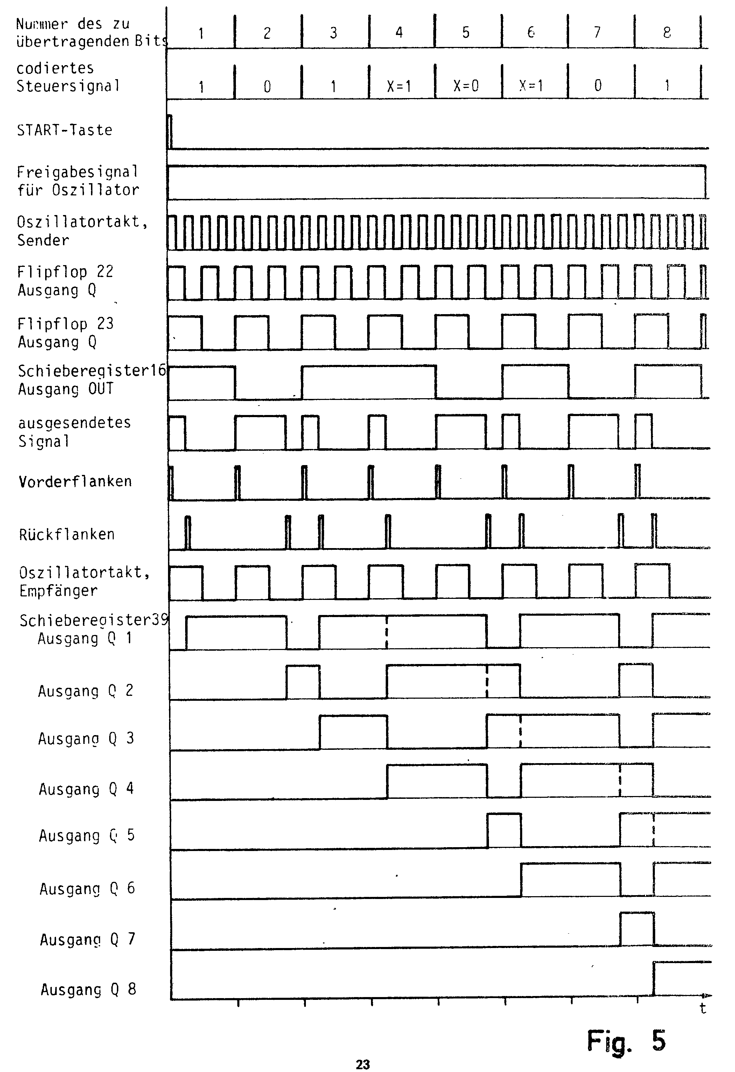

In Fig. 4 ist die Schaltung des Empfängerteils dargestellt. Tritt ein Magnetfeld ausreichender Intensität im Bereich des Reed-Kontaktes 13 auf, so schließt dieser und verbindet den Eingang eines Inverters 31 der während der übrigen Zeit über einen Widerstand R 12 mit dem H-Potential (+) verbunden ist, mit dem L-Potential, so daß der Ausgang des Inverters von L auf H geht. Ein Kondensator C 6 dient zur Beseitigung von bei einem eventuellen Prellen des Kontaktes auftretenden Störimpulsen. Aus der ansteigenden Flanke des Ausgangssignals des Inverters 31 wird mittels des aus einem Kondensator C 7 und einem Widerstand R 13 bestehenden Differenziergliedes ein H-Impuls gebildet, der einen Zähler 32 über seinen »RESET«-Eingang zurücksetzt. Der selbe Impuls wird mittels eines zusätzlichen Inverters 33 negiert und setzt den Ausgang eines NAND-Gatters 34 auf H, woraufhin der aus einem NAND-Gatter 35, einem Inverter 36, sowie Widerständen R 14, R 15 und einem Kondensator C 8 gebildete Oszillator startet. Das NAND-Gatter 34 stellt zusammen mit einem NAND-Gatter 37 eine bistabile Schaltung dar, so daß sein Ausgang weiterhin auf dem H-Pegel verbleibt. Die Frequenz des Oszillators wird durch die angeschlossenen passiven Bauelemente und die Versorgungsspannung der Energiequelle festgelegt. Eine Nachstimmung eines derartigen, die Auswertung der empfangenden Signale bestimmenden Oszillators ist mit einem relativ großen technischen Aufwand verbunden und erfordert zusätzliche Vergleichs- und Regelmittel. Die Regelung auf eine Sollfrequenz nimmt stets einen gewissen Zeitraum in Anspruch, währenddessen noch keine Signalübertragung erfolgen kann und es besteht die Gefahr der Ausbildung von Regelschwingungen.

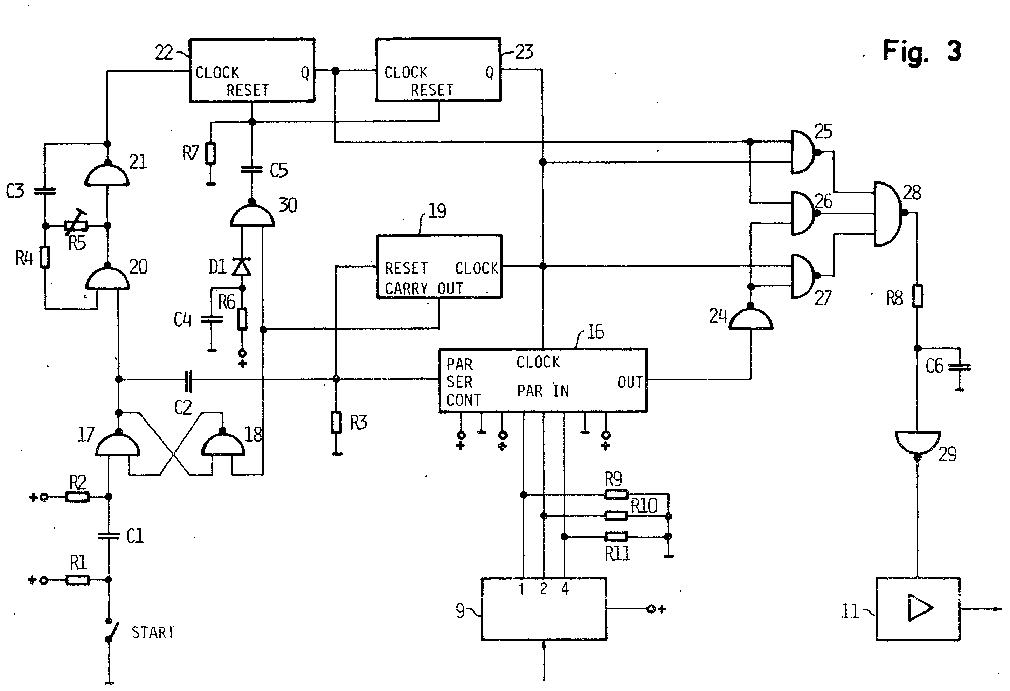

Die Taktfrequenz des Oszillators ist um den Faktor $2^{10}$ höher als die Taktfrequenz bei der Signalübertragung. Die Oszillatorfrequenz wird deshalb einem Binär-Zähler 32 zugeführt, der einen Frequenzteiler bildet und an dessen Ausgängen Rechteck-Signale anliegen, deren Frequenz gegenüber der Eingangsfrequenz um eine Potenz von 2 herabgesetzt ist und die ein Taktverhältnis von 1 : 1 aufweisen. Am Ausgang Q 10 des Zählers ergibt sich ein Signal, welches demjenigen am Ausgang des Flip-Flops 23 in Fig. 3, allerdings in negierter Form, entspricht, wobei die Synchronität von Sender- und Empfängertaktsignal vorausgesetzt ist. Da diese Synchronität aber aus den genannten Gründen nicht von vornherein gegeben ist, wird der Zähler 32 beim Eintreffen der vorderen Flanke eines Informations-Bits des übertragenen Steuersignals auf null zurückgesetzt. Der freiliegende Oszillatortakt bewirkt, daß der Zähler 32 jedesmal jeweils wieder von null beginnend aufwärts zählt. Nach 512 Impulsen geht das Ausgangssignal Q 10 des Zählers 32 in den anderen logischen Zustand über, so daß sich bei der Signalauswertung der andere Abfragezustand einstellt. Vorher festgestellte Rückflanken des Eingangssignals wurden als »1« gewertet, während von jetzt ab auftretende Rückflanken als »0« ausgewertet werden. Nach weiteren 512 Impulsen oder nach einem durch den Reed-Kontakt 13 ausgelösten RESET, geht der Ausgang Q 10 wieder in seinen Anfangszustand zurück. Auf diese Weise ist auch bei größeren Abweichungen des Oszillatortaktes im Empfängerteil von demjenigen im Senderteil eine Synchronität bei der Signalauswertung gewährleistet.

Das Ausgangssignal an Q 10 des Zählers 32 wird mittels eines invertierenden Gatters, hier des NOR-Gatters 38, negiert und dem seriellen Dateneingang eines Schieberegisters 39 zugeführt. Das resultierende Empfängertaktsignal ist in Zeile 12 der Impulsdarstellungen in Fig. 5 wiedergegeben. Die vorhergehenden Zeilen 10 und 11 zeigen die Impulse, welche von der Vorder- bzw. Rückflanke der über den Reed-Schalter 13 aufgenommenen Signale gebildet werden. Die den Rückflanken zugeordneten Impulse werden mittels eines Inverters 40 und eines aus einem Kondensator C 9 und einem Widerstand R 16 bestehenden Differenziergliedes erzeugt. Diese Impulse »takten« das Schieberegister 39. Hierdurch wird eine gut funktionierende und einfach zu realisierende Serien-/Parallelumsetzung für das empfangene Signal geschaffen, da jeweils auf die Rückflanke der Eingangsimpulse hin die am Eingang »DATA IN« des Schieberegisters 39 vorhandene Information in dieses eingespeichert und die bereits vorhandenen Informationen um eine Position weitergerückt werden.

Da der weitere Eingang des NOR-Gatters 38 mit dem Ausgang Q 11 einer weiteren nachgeschalteten Frequenzteilerstufe des Zählers 32 verbunden ist, verzögert sich die Rückkehr in den Abfragezustand »1« für die Rückflanken bei der Signalauswertung. Das hat die vorteilhafte Folge, daß, auch wenn die Zeitkonstanten der zeitbestimmenden Elemente des Empfängers stark verkleinert sind, der volle Zeitraum bis zum Erscheinen des nächsten Referenz-Signals zur Auswertung zur Verfügung steht.

Für die Decodierung des aufgenommenen Signals ist es im wesentlichen gleichbedeutend, wenn die Eingangssignale an den Eingängen »DATA IN« und »CLOCK« vertauscht sind. In einem Fall wird die zeitliche Lage der das weitere Signal bildenden Rückflanke im Empfänger dadurch ermittelt, daß beim Auftreten der Rückflanke der logische Zustand des Empfängertakt-Signals festgehalten wird. Im anderen Fall wird zum Zeitpunkt des Wechsels des logischen Zustands des Empfängertaktes der Zustand des Sendertaktes eingespeichert. In beiden Fällen enthält das Empfängertakt-Signal die zur Auswertung notwendigen Zeitschranken.

An den Ausgängen Q 1 bis Q 8 des Schieberegisters entsteht beim Eintakten des Signals der in den unteren Zeilen von Fig. 5 dargestellte Signalverlauf. Es liegt — nachdem das vollständige Signal eingegeben wurde — eine Anzahl von logischen Signalen an, welche in ihrer Folge dem ursprünglichen codierten Steuersignal entsprechen. Mittels der aus den NAND-Gattern 41 und 42, dem Invertern 43 und 45 und den NOR-Gliedern 44 und 46 bestehenden logischen Schaltung wird festgestellt, ob die redundanten, im Steuersignal mit übertragenen Bits die zum Auslösen des Steuervorgangs erforderliche Konfiguration aufweisen. Ist das der Fall, so geht der Ausgang des NOR-Gatters 46 in den logischen H-Zustand. Mittels eines Kondensators C 10 und eines Widerstandes R 17 wird daraus ein Impuls geformt, der bewirkt, daß die an den Ausgängen Q 3 bis Q 5 anliegenden Signale in einen Speicher 47 übertragen werden. Mit diesem verbundene Gatter können in vorteilhafter Weise in einer logischen Schaltung zusammengefaßt sein, die ein bestimmtes Ausgangssignal abgibt, wenn ein vorgegebenes logisches Eingangssignal in Kombination an den Eingängen vorhanden ist. Hierbei ist vorausgesetzt, daß die zusammengefaßten, mit dem Ausgang des Inverters 31 verbundenen Eingänge der NOR-Gatter 51 bis 53 den L-Zustand einnehmen. Mittels eines Decoders 48 wird in Abhängigkeit von den im Speicher festgehaltenen Signalzuständen einer der Widerstände R 18 bis R 25 mit dem Ausgang »COMMON« des Decoders verbunden. Dieser Ausgang ist wie die zusammengefaßten anderen Anschlüsse der Widerstände R 18 bis R 25 mit der Schaltung des Schrittmachers verbunden, dessen Betriebsparameter verändert werden sollen (Pfeile 49 und 50). Die Widerstände können dabei beispielsweise als frequenzbestimmende Glieder für einen die Stimulationsimpulse des Schrittmachers erzeugenden Oszillator dienen.

Wird kein Impuls mehr empfangen — sei es daß das vollständige Signal eingetroffen ist oder daß ein fehlerhaftes bzw. unvollständiges Signal empfangen wurde — so zählt der Zähler 32 solange weiter bis der Ausgang Q 12 den H-Zustand einnimmt und damit $2^{11}$ Impulse gezählt wurden. Mittels eines Kondensators C 11 und eines Widerstandes R 26 wird aus dem Signal am Ausgang Q 12 ein Impuls geformt, welcher das Schieberegister 39 zurücksetzt und, negiert durch einen Inverter 51, über das NAND-Gatter 37 den aus den NAND-Gattern 35 und 36 bestehenden Oszillator stoppt.

Durch die Position der redundanten Bits am Anfang und Ende des zu übertragenden Steuersignals werden Fehlsteuerungen verhindert, falls durch Störsignale Impulse wegfallen oder durch entstehende Einbrüche in Impulse das Vorhandensein zusätzlicher Impulse vorgetäuscht wird, da nämlich ein Zurücksetzen des Zählers 32 jedesmal erfolgt, wenn eine Vorderflanke ermittelt wird. Durch fehlende oder vorgetäuschte zusätzliche Impulse, die durch äußere Störeinflüsse auch vor Beginn bzw., anschließend an die eigentliche Übertragung des Steuersignals entstehen können, verschiebt sich stets die Position der redundanten Bits im empfangenden und decodierten Signalverlauf, der an den Ausgängen Q 1 bis Q 8 des Schieberegisters 39 anliegt. Wenn die redundanten Bits — wie bei dem dargestellten Ausführungsbeispiel — eine Folge von aufeinanderfolgend unterschiedlichen logischen Signalen bilden, bewirkt das Fehlen eines oder das Entstehen eines zusätzlichen Impulses bei der Signalübertragung stets eine Veränderung in den Positionen der redundanten Bits. Vorausgesetzt ist eine Signalverschiebung um eine Position, was für die weitaus meisten Störungsfälle zutreffend sein wird. Ist die Signalübertragung stärker gestört, so besteht die Gefahr, daß zufällig Störsignale oder Anteile des Nutzsignals, die in die Position der redundanten Bits gelangen, genau deren Konfiguration haben, was allein zu einer Fehlsteuerung führen würde — die Wahrscheinlichkeit dafür ist jedoch sehr gering. Dadurch, daß die redundanten Bits bei der Signalübertragung eine das Nutzsignal umgebende Stellung einnehmen, ist auch eine große Wahrscheinlichkeit dafür gegeben, daß, wenn diese ungestört blieben, auch die Nutzsignale nicht von einer Störung betroffen sind.

Bevor die zur kurzfristigen Umschalten des Schrittmachers auf eine charakteristische Frequenz zu Prüfzwecken dienenden Schaltungsteile im einzelnen beschrieben werden, soll zunächst deren grundsätzliche Funktion anhand der in den Fig. 6 und 6a wiedergegebenen Blockdarstellungen näher erläutert werden.

In Fig. 6 ist ein Blockdiagramm der ersten Ausführungsform der Schaltung wiedergegeben. Ein programmierbarer Datenspeicher 61 (der in seiner Funktion dem Schieberegister 39 entsprechen kann) wird durch nicht dargestellte Mittel für die Einstellung der jeweiligen permanenten Betriebsweise des Herzschrittmachers von außen über ein Magnetfeld angesteuert, wie es durch den Eingangspfeil angedeutet ist. Ein Bauelement 62 enthält diejenigen Schaltzustände gespeichert, welche zum Umschalten des frequenzbestimmenden Elementes 63 auf die charakteristische Frequenz des Herzschrittmachers erforderlich sind. Durch Anlegen eines Permanentmagnetfeldes wird ein Umschaltelement 64 für Logiksignale so gesteuert, daß das frequenzbestimmende Element für den Zeitraum des Vorhandenseins des Permanentmagnetfelds nicht mehr mit dem programmierbaren Datenspeicher 61, sondern mit dem Bauelement 62 verbunden ist. Wichtig ist dabei, daß durch das Permanentmagnetfeld der Inhalt des Datenspeichers 61 nicht verändert wird. Das frequenzbestimmende Element 63 (Pfeil) steuert in Abhängigkeit von den ihm zugeführten logischen Schaltzuständen die Rate der Anregungsimpulse des Schrittmachers.

In Fig. 6a ist ein Blockdiagramm der zweiten Ausführungsform der erfindungsgemäßen Schaltung wiedergegeben. Hierbei sind durch ein digitales Signal auswählbare, jeweils durch Verbinden mit einem die Stimulationsimpulse erzeugenden Oszillator 69 die Herzschrittmacherfrequenz bestimmende Elemente 65 an einen programmierbaren Datenspeicher 66 angeschlossen, wie bei dem in Fig. 6 dargestellten Ausführungsbeispiel der programmierbare Datenspeicher 61, durch das Steuersignal beeinflußbar ist, wodurch er jeweils eines der frequenzbestimmenden Elemente 65 auswählt. Ein Umschalter 67, der entsprechend dem Umschalter 64 in Fig. 6 durch ein zeitweise anliegendes permanentes Magnetfeld betätigt wird, verbindet für die Zeitdauer des Vorhandenseins dieses Feldes ein frequenzbestimmendes Element 68 mit den Impulserzeugermitteln des Herzschrittmachers, d. h. dem Oszillator 69. Hierbei bleibt ebenfalls der Inhalt des Datenspeichers 66 unverändert.

Es folgt jetzt die Fortsetzung der Beschreibung von Fig. 4. Für diejenigen Zeiten, zu denen der Ausgang des Inverters 31 sich im H-Zustand befindet, der Reed-Schalter also infolge eines vorhandenen Magnetfeldes geschlossen ist, nehmen die Ausgänge der NOR-Gatter 51a bis 53 den L-Zustand ein. Hierdurch wird der übrige Programmiervorgang nicht gestört. Da bei impulsförmigem äußeren Magnetfeld dieser Zustand nur kurzzeitig eintritt, wird einerseits die Impulsabgabe des Schrittmachers kaum und andererseits der Einspeichervorgang des Schieberegisters 39 überhaupt nicht beeinflußt. Wird kein Impuls mehr empfangen, d. h. ist die Programmierung abgeschlossen und das äußere Magnetfeld abgeschaltet oder wird, zwecks Einstellung der charakteristischen Frequenz, ein Dauermagnetfeld aufrechterhalten, so zählt der Zähler 32 solange weiter bis der Ausgang Q 12 den H-Zustand einnimmt und damit $2^{11}$ Impulse empfangen wurden. Mittels eines Kondensators C11 und eines Widerstandes R 26 wird aus dem Signal am Ausgang Q 12 ein Impuls geformt, welcher das Schieberegister 39 zurücksetzt und, negiert durch einen Inverter 51, über das NAND-Gatter 37 den aus den NAND-Gattern 35 und 36 bestehenden Oszillator stoppt. Eine Veränderung des Inhalts des Speichers 47 erfolgt nicht, daß die redundanten Bits nicht vorhanden sind bzw. in einer ausschließlich Nullen aufweisenden Konfiguration ausgegeben werden.

Beim Einschalten der charakteristischen Frequenz des Schrittmachers zu Prüfzwecken durch Annäherung eines Dauermagneten an den implantieren Schrittmacher wird das äußere Dauermagnetfeld über den zur Prüfung erforderlichen Zeitraum aufrechterhalten, wobei die Ausgänge der NOR-Gatter 51a bis 53 den L-Zustand beibehalten. Über den Decoder 48 wird dieses die Konfiguration »000« darstellende Signal in der Weise ausgewertet, daß die frequenzbestimmende Elemente bildende Widerstände R 18 bis R 25 so geschaltet werden, daß der Schrittmacher mit der charakteristischen Frequenz arbeitet. Voraussetzung bei dieser Schaltung ist, daß — wie dargelegt — auch bei der Frequenzbeeinflussung durch Programmierung die für Prüfzwecke gewählte charakteristische Frequenz mittels eines Signals mit einer Zeichenfolge einstellbar ist, wie sie durch logische Schaltungen (NOR-Gatter 51a bis 53) beim Auftreten des Dauermagnetfeldes bestimmt wird. (Beim hier beschriebenen Beispiel ist das die Ziffernfolge »000«).

Bei der in Fig. 4a dargestellten Ausführungsform der Erfindung, die der in Fig. 6a prinzipiell dargestellten entspricht, brauchen im Vergleich zu der Schaltung gemäß Fig. 4 nur wenige Bauelemente geändert zu werden. Anstelle der NOR-Gatter 51 bis 53 ist ein Umschalter 54 eingefügt, der statt der mit der Decodierschaltung 48 verbundenen die Frequenz bestimmenden Elemente bildende Widerstände R 18 bis R 25 einen weiteren Widerstand R 26 einschaltet, der den Oszillator des Schrittmachers auf die zu Prüfzwecken erforderliche Impulsrate umschaltet. Der Umschalter 64 kann dabei aus bekannten elektronischen Schaltern aufgebaut sein, die bezüglich ihrer Technologie den übrigen verwendeten Bauelementen entsprechend ausgewählt sind.

Bei dieser zuletzt dargestellten Ausführungsform wird die Decodierungsvorrichtung 48 umgangen und es ist nicht erforderlich, daß die charakteristische Frequenz in den programmierbaren Frequenzen enthalten ist.

Die dargestellten Ausführungsbeispiele stellen nur einige der Realisierungsmöglichkeiten der Erfindung dar. Es ist eine große Anzahl Varianten denkbar, die insbesondere durch die Art der durch die Programmierung zu beeinflussenden Schrittmacherfunktionen und die zur Verwendung gelangenden Bauelemente bedingt sind.

**Patentansprüche**

1. Sender-Empfänger-System zum Aussenden und Empfangen eines digitalen Steuersignals, wobei sich der Sender außerhalb des Körpers eines Patienten und der Empfänger in einem in dem Patienten implantierten Herzschrittmacher befindet, zwecks Einschaltung eines ausgewählten Betriebszustandes mittels des digitalen Steuersignals, und wobei das digitale Steuersignal und ein zusätzliches Kontrollsignal seriell vom Sender zum Empfänger übertragen und die Umschaltung des Betriebszustands nur dann freigegeben wird, wenn das zusätzliche Kontrollsignal korrekt übermittelt ist, dadurch gekennzeichnet,

daß im Sender Mittel zur Umwandlung des digitalen Steuersignals in ein auszusendendes Signal in der Weise vorgesehen sind, daß in einer Folge von Referenzsignalen (Vorderflanken 1, 2) jeweils auf ein derartiges Referenzsignal hin ein in Bezug dazu in seiner zeitlichen Lage veränderbares weiteres Signal (Rückflanken 3, 4, 4') folgt, dessen zeitliche Position von einem Anteil der in dem digitalen Steuersignal enthaltenen Information bestimmt ist,

daß der Empfänger zur Wiedergewinnung des Steuersignals Mittel zur Auswertung der empfangenen Signale in der Weise enthält, daß jeweils die zeitliche Lage des weiteren Signals (3, 4, 4') in Bezug auf mindestens ein eine Zeitschranke (5, 6) bildendes Signal ermittelt wird, das in einem durch Zeitgebermittel des Empfängers festgelegten zeitlichen Abstand auf das empfangene Referenzsignal (1, 2) folgt, und daß das Kontrollsignal einen redundanten Signalanteil des zu übertragenden Steuersignals bildet und mit diesem gemeinsam ausgewertet wird.

2. System nach Anspruch 1, dadurch gekennzeichnet, daß das Steuersignal binär codiert ist und daß eine von zwei zeitlichen Lagen des weiteren Signals (3, 4, 4') jeweils von einem Bit des Steuersignals festgelegt wird.

3. System nach einem der Ansprüche 1 oder 2, dadurch gekennzeichnet, daß das digital codierte Signal in serieller Form eine Anzahl von Referenz- bzw. weiteren Signalen aufweist und ein Umschalten in den ausgewählten, durch das aus diesen Signalen wiedergewonnene Steuersignal bestimmten Betriebszustand erfolgt, sobald den Empfänger nach einem Anfangszustand die Anzahl von Signalen erreicht hat.

4. System nach einem der Ansprüche 1 bis 3, dadurch gekennzeichnet, daß ein im Empfänger erzeugtes logisches Signal (32, Q 10) im durch die Zeitschranke definierten Zeitpunkt seinen Zustand ändert und daß logische Mittel (Schieberegister 39) vorgesehen sind, die auf das vom Empfänger aufgenommene weitere Signal (3, 4, 4') hin den augenblicklichen Zustand des im Empfänger erzeugten logischen Signals festhalten.

5. System nach einem der Ansprüche 1 bis 3, dadurch gekennzeichnet, daß das weitere Signal aus einer zeitlichen Änderung des logischen Zustands des ausgesendeten Signals besteht.

6. System nach den Ansprüchen 1, 2 und 5, dadurch gekennzeichnet, daß im Empfänger Mittel zum Festhalten des logischen Zustands im durch die Zeitschranke definierten Zeitpunkt vorgesehen sind.

7. System nach einem der vorangehenden Ansprüche, dadurch gekennzeichnet, daß der zeitliche Abstand des die Zeitschranken bildenden Signals von den empfangenen Referenzsignalen (1, 2) so gewählt ist, daß bei einer durch Änderung der zeitbestimmenden Elemente (C 8, R 14, R 15) des Empfängers infolge vorhersehbarer Alterung bedingten Veränderung dieses zeitlichen Abstands das die Zeitschranke bildende Signal während der vorgesehenen Betriebsdauer des Herzschrittmachers nicht mit dem empfangenen weiteren Signal (3, 4, 4') zusammenfällt.

8. System nach Anspruch 7, dadurch gekennzeichnet, daß bei einer im wesentlichen gleichen Tendenz der durch Änderung der zeitbestimmenden Elemente (C 8, R 14, R 15) des Empfängers bedingten Veränderung des zeitlichen Abstands des die Zeitschranke bildenden Signals von dem empfangenen Referenzsignal (1, 2) die zeitbestimmenden Elemente des Empfängers so dimensioniert sind, daß die Zeitschranke bei Inbetriebnahme des Herzschrittmachers von demjenigen empfangenen weiteren Signal (3, 4, 4') einen kleinen zeitlichen Abstand aufweist, von dem es sich mit der Alterung der zeitbestimmenden Elemente entfernt.

9. System nach einem der vorangehenden Ansprüche, dadurch gekennzeichnet, daß Mittel vorgesehen sind, um den Empfänger in den vor Erscheinen des Signalzugs bestehenden Ausgangszustand zurückzusetzen, wenn das weitere Signal (3, 4, 4') nicht vor einem nachfolgenden Referenzsignal (2) erscheint.

10. System nach einem der vorangehenden Ansprüche, dadurch gekennzeichnet, daß Mittel vorgesehen sind, um den Empfänger in den vor Erscheinen des Signalzugs bestehenden Ausgangszustand zurückzusetzen, wenn ein nachfolgendes Referenzsignal (2) nicht innerhalb eines durch zeitbestimmende Elemente des Empfängers festgelegten Zeitraums erscheint.

11. System nach einem der vorangehenden Ansprüche, dadurch gekennzeichnet, daß Referenzsignale jeweils die Vorder- (1, 2) und die weiteren Signale jeweils die Rückflanke (3, 4, 4') von Impulsen bilden, die in dem ausgesendeten Signal enthalten sind, oder umgekehrt.

12. System nach einem der vorangehenden Ansprüche, dadurch gekennzeichnet, daß das Maß der Beeinflussung der zeitlichen Lage des weiteren Signals (3, 4') von einem Anteil der in dem digital codierten Steuersignal enthaltenen Information im Sender zum Ausgleich der Veränderungen zeitbestimmender Elemente des Empfängers variierbar ist.

13. System nach einem der vorangehenden Ansprüche, dadurch gekennzeichnet, daß im Empfänger ein bei mindestens einem vorgegebenen Zählerstand Zeitschranken darstellende Signale auslösender Zähler (32) vorgesehen ist, der jeweils durch das Auftreten eines nachfolgenden Referenzsignals (2) in einen Ausgangszustand zurückgesetzt wird.

14. System nach Anspruch 13, dadurch gekennzeichnet, daß der von einem Oszillator (35, 36) angesteuerte Zähler (32) aus einem Frequenzteiler besteht, wobei zur Unterscheidung von zwei verschiedenen zeitlichen Lagen des weiteren Signals (3, 4, 4') eine Zeitschranke durch die Änderung des logischen Zustands des Ausgangssignals des Frequenzteilers gebildet wird.

15. System nach den Ansprüchen 14 und 4, dadurch gekennzeichnet, daß der Ausgang des Frequenzteilers mit dem Dateneingang (DATA IN) eines Schieberegisters (39) verbunden ist, wobei beim Auftreten des weiteren Signals (3, 4, 4') jeweils ein Impuls an dessen Takteingang (CLOCK) gelangt.

16. System nach Anspruch 10 und 13, dadurch gekennzeichnet, daß das Zurücksetzen durch ein Signal erfolgt, das durch den Zähler (32) bei einem vorgegebenen Zählerstand ausgelöst wird.

17. System nach einem der vorangehenden Ansprüche, dadurch gekennzeichnet, daß im Sender Mittel zum vorübergehenden Einstellen einer Impulsfrequenz für Meßzwecke bei einem mittels eines äußeren Magnetfelds über einen eingebauten Magnetschalter (13) programmierbaren künstlichen Herzschrittmacher vorgesehen sind, wobei die sich tatsächlich einstellende Impulsfrequenz für mindestens einen Betriebswert, wie beispielsweise die Versorgungsspannung, charakteristisch ist, und im Empfänger Mittel, die, gesteuert durch den Magnetschalter (13), die Impulsfrequenz des Schrittmachers auf die Impulsfrequenz für Meßzwecke umschalten, solange das äußere Magnetfeld aufrechterhalten bleibt, wobei das zu dem zuletzt eingestellten ausgewählten Betriebszustand des Schrittmachers gehörige Steuersignal gespeichert erhalten bleibt.

18. System nach Anspruch 17, dadurch gekennzeichnet, daß Mittel (13, 31, 51a, 52, 53) vorgesehen sind, welche für die Zeitdauer der Aufrechterhaltung des Magnetfelds einer mindestens ein frequenzbestimmendes Element (Widerstände R 18 bis R 25) ansteuernden, im übrigen von dem digitalen Steuersignal beeinflußten Decodierungsschaltung (48) ein Signal zuführen, welches die Decodierungsschaltung (48) dazu veranlaßt, zeitweise die Impulsfrequenz für Meßzwecke einzustellen.

19. System nach Anspruch 18, dadurch gekennzeichnet, daß Mittel (51a, 52, 53, 48) vorgesehen sind, um die Impulsfrequenz für Meßzwecke dadurch zeitweise einzuschalten, daß mindestens ein frequenzbestimmendes Element (Widerstände R 18 bis R 25) in seinem Wert verändert und/oder ein anderes Element (Widerstand R 26) an seiner Stelle eingeschaltet wird.

## Claims

1. Transmitter-receiver-system for the transmission and reception of a digital control signal, in which the transmitter is located outside the body of a patient and the receiver is located in a heart pacemaker implanted in the patient, for the purpose of switching-in a selected operating condition by means of the digital control signal, and in which the digital control signal and an additional check signal are serially transferred from the transmitter to the receiver and the change-over of the operating condition is only permitted if the additional check signal is transmitted correctly, characterised in that means are provided in the transmitter for the conversion of the digital control signal into a signal to be transmitted in such a way that in a sequence of reference signals (leading flanks 1, 2) a further signal (trailing flanks 3, 4, 4') follows as a result of each such reference signal, the position in time of the further signal being variable with reference to the reference signal and being determined by a part of the information included in the digital control signal, that, to reproduce the control signal, the receiver includes means for evaluating the received signals in such a way that there is transmitted in each case the position in time of the further signal (3, 4, 4') with reference to a signal forming a time barrier (5, 6) and following the received reference signal (1, 2) by a separation in time which is defined by timing means of the receiver, and that the check signal forms a redundant signal component of the control signal to be transferred and is evaluated jointly with the latter.

2. System according to claim 1, characterised in that the control signal is binary coded and that one of two positions in time of the further signal (3, 4, 4') is determined in each case by one bit of the control signal.

3. System according to any one of claims 1 or 2, characterised in that the digitally coded signal comprises in serial form a number of reference and further signals, and a change-over results in the selectred operating condition, which is determined by the control signal reproduced from these signals, as soon as the receiver has reached the number of signals after an initial state.

4. System according to any one of claims 1 to 3, characterised in that a logic signal (32, Q 10) produced in the receiver changes its state at the point in time defined by the time barrier, and that logic means (shift register 39) are provided which, as a result of the further signal (3, 4, 4') picked up by the receiver, retain the instantaneous state of the logic signal produced in the receiver.

5. System according to any one of claims 1 to 3, characterised in that the further signal comprises a temporal modification of the logic state of the transmitted signal.

6. System according to claims 1, 2 and 5, characterised in that means are provided in the receiver for retaining the logic state at the point in time defined by the time barrier.

7. System according to any one of the preceding claims, characterised in that the spacing in time of the signal forming the time barrier from the received reference signals (1, 2) is so chosen that, upon a change of this spacing in time caused by variation of the time-determining elements (C 8, R 14, R 15) of the receiver as a result of forseeable ageing, the signal forming the time barrier does not coincide with the received further signal (3, 4, 4') during the envisaged working life of the heart pacemaker.

8. System according to claim 7, characterised in that upon a change in essentially the same tendency of the spacing in time of the signal forming the time barrier from the received reference signal (1, 2) caused by variation of the time-determining elements (C 8, R 14, R 15) of the receiver, the time-determining elements of the receiver are so dimensioned that when putting the heart pacemaker into operation the time barrier has a small spacing in time from that received further signal (3, 4, 4') from which it moves away during ageing of the time-determining elements.

9. System according to any one of the preceding claims, characterised in that means are provided which, when the further signal (3, 4, 4') does not appear before a following reference signal (2), reset the receiver to its initial state existing before appearance of the signal train.

10. System according to any one of the preceding claims, characterised in that means are provided which, when a following reference signal (2) does not appear within a period of time defined by timedetermining elements of the receiver, reset the receiver to its initial state existing before appearance of the signal train.

11. System according to any one of the preceding claims, characterised in that reference signals constitute the respective leading flanks (1, 2) and the further signals the respective trailing flanks (3, 4, 4') of pulses which are contained in the transmitted signal, or vice versa.

12. System according to any one of the preceding claims, characterised in that the amount of the influence of the position in time of the further signal (3, 4') by a part of the information in the transmitter in the digitally coded control signal is variable for compensating the changes in time-determining elements of the receiver.

13. System according to any one of the preceding claims, characterised in that in the receiver there is provided a counter (32) which emits signals representing time barriers at at least one predetermined counting state, the counter being reset to an initial state by the appearance of each following reference signal (2).

14. System according to claim 13, characterised in that the counter (32) triggered by an oscillator (35, 36) comprises a frequency divider, and to distinguish between two different positions in time of the further signal (3, 4, 4') a time barrier is formed by the variation of the logic state of the output signal of the frequency divider.

15. System according to claims 14 and 4, characterised in that the output of the frequency divider is connected to the data input (DATA IN) of a shift register (39), and upon the appearance of the further signal (3, 4, 4') a pulse arrives in each case at the clock input (CLOCK) thereof.

16. System according to claims 10 and 13, characterised in that the resetting results form a signal which is emitted by the counter (32) at a predetermined counting state.

17. System according to any one of the preceding claims characterised in that there are provided means in the transmitter for the temporary adjustment of a pulse frequency for measurement purposes in an artificial heart pacemaker programmable by means of an external magnetic field via a built-in magnetic switch (13), the actual self-adjusting pulse frequency being characteristic of at least one operating parameter, for example the supply voltage, and means in the receiver which controlled by the magnetic switch (13), change over the pulse frequency of the pacemaker to the pulse frequency for measurement purposes as long as the external magnetic field is maintained, and the control signal remains stored which appertains to the finally adjusted selected operating condition of the pacemaker.

18. System according to claim 17, characterised in that means (13, 31, 51a, 52, 53) are provided which for the period of time during which the magnetic field is maintained, delivers, to a decoding circuit (48) which controls at least one frequency determining element (resistors R 18 to R 25) and besides is influenced by the digital control signal, a signal which causes the decoding circuit (48) additionally to adjust temporarily the pulse frequency for measurement purposes.

19. System according to claim 18, characterised in that means (51a, 52, 53, 48) are provided to temporarily switch in the pulse frequency for measurement purposes by varying the value of at least one frequencydetermining element (resistors R 18 to R 25) and/or switching in another element (resistor R 26) in its place.

**Revendications**

1. Système émetteur-récepteur pour émettre et recevoir un signal de commande numérique, dont l'émetteur est situé à l'extérieur du corps d'un patient et dont le récepteur est situé dans un stimulateur cardiaque implanté chez le patient, en vue de l'enclenchement d'un mode de fonctionnement choisi au moyen du signal de commande numérique, dans lequel le signal de commande numérique et un signal de contrôle supplémentaire sont transmis en série de l'émetteur au récepteur et dans lequel la commutation au nouveau mode de fonctionnement est seulement autorisée lorsque le signal de contrôle supplémentaire a été transmis convenablement, caractérisé en ce

que l'émetteur comprend des moyens pour convertir le signal de commande numérique en un signal à émettre qui contient une série de signaux de référence (flancs de montée 1, 2), dont l'un est suivi chaque fois d'un signal supplémentaire (flancs arrière 3, 4, 4') dont la position dans le temps, par rapport à ce signal de référence, est variable et est fixée par une portion de l'information contenue dans le signal de commande numérique,

que le récepteur, pour extraire le signal de commande du signal reçu, comprend des moyens d'exploitation du signal reçu, qui déterminent chaque fois la position dans le temps du signal supplémentaire (3, 4, 4') par rapport à au moins un signal constituant une limite de temps (5, 6), lequel suit le signal de référence (1, 2) reçu à un intervalle de temps fixé par des moyens de minutage prévus dans le récepteur, et

que le signal de contrôle est constitué par une portion redondante du signal de commande à transmettre et est exploité ensemble avec celui-ci.

2. Système selon la revendication 1, caractérisé en ce que le signal de commande est codé binaire et en ce que l'une de deux positions dans le temps du signal supplémentaire (3, 4, 4') est fixée chaque fois par un bit du signal de commande.

3. Système selon la revendication 1 ou 2, caractérisé en ce que le signal codé en numérique présente, sous une forme sérielle, un certain nombre de signaux de référence respectivement de signaux supplémentaires et que la commutation au mode de fonctionnement choisi, déterminée par le signal de commande extrait de ces signaux, s'effectue dès que, à la suite d'un état initial, ledit nombre de signaux a atteint le récepteur.

4. Système selon une revendications 1 à 3, caractérisé en ce qu'un signal logique (32, Q 10) produit dans le récepteur change d'état au moment fixé par la limite de temps et en ce que des moyens logiques (registre à décalage 39) sont prévus qui retiennent à la suite de la réception par le récepteur du signal supplémentaire (3, 4, 4') le signal logique produit audit moment dans le récepteur.

5. Système selon une des revendication 1 à 3, caractérisé en ce que le signal supplémentaire consiste en un changement dans le temps de l'état logique du signal émis.

6. Système selon les revendications 1, 2 et 5, caractérisé en ce que des moyens sont prévus dans le récepteur pour retenir l'état logique au moment défini par la limite de temps.

7. Système selon une des revendications précédentes, caractérisé en ce que l'intervalle dans le temps entre le signal formant les limites de temps et les signaux de référence reçus (1, 2) est choisi de telle manière que, en cas de changement de cet intervalle par suite d'un changement par vieillissement prévisible des éléments de détermination de temps (C 8, R 14, R 15) du récepteur, le signal constituant la limite de temps ne peut pas coïncider avec le signal supplémentaire (3, 4, 4') reçu pendant la durée de service prévue du stimulateur cardiaque.

8. Système selon la revendication 7, caractérisé en ce que, au cas où la tendance du changement de l'intervalle entre le signal constituant la limite de temps et le signal de référence (1, 2) reçu par suite d'un changement des éléments de détermination de temps (C 8, R 14, R 15) du récepteur reste sensiblement la même, les éléments de détermination de temps du récepteur sont dimmensionnés de telle manière que la limite de temps se trouve à une petite distance dans le temps dudit signal supplémentaire (3, 4, 4') à la mise en service du stimulateur cardiaque et s'éloigne de ce signal à mesure que les éléments de détermination de temps vieillissent.

9. Système selon une des revendications précédentes, caractérisé en ce que des moyens sont prévus pour ramener le récepteur à l'état de départ existant avant l'apparition du train de signaux lorsque le signal supplémentaire (3, 4, 4') n'apparaît pas avant un signal de référence (2) suivant.

10. Système selon une des revendications précédentes, caractérisé en ce que des moyens sont prévus pour ramener le récepteur à l'état de départ existant avant l'apparition du train de signaux lorsqu'un signal de référence (2) suivant n'apparaît pas à l'intérieur d'un laps de temps fixé par des éléments de détermination de temps du récepteur.

11. Système selon une des revendications précédentes, caractérisé en ce que des signaux de référence sont constitués chacun par un flanc de montée (1, 2) et les autres signaux sont constitués chacun par un flanc arrière (3, 4, 4') d'impulsions contenues dans le signal émis, ou inversement.

12. Système selon une des revendications précédentes, caractérisé en ce que l'étendue du changement possible de la position dans le temps du signal supplémentaire (3, 4') est variable, dans l'émetteur, pour compenser les changements d'éléments de détermination de temps du récepteur, par une portion de l'information contenue dans le signal de commande numérique.

13. Système selon une des revendications précédentes, caractérisé en ce que le récepteur contient un compteur (32) qui produit des signaux représentant des limites de temps à au moins une position de comptage préfixée et qui est remis chaque fois à une position de départ par l'apparition consécutive d'un signal de référence (2).

14. Système selon la revendication 13, caractérisé en ce que le compteur (32), commandé par un oscillateur (35, 36), constitue un diviseur de fréquence et en ce que le changement de l'état logique du signal de sortie de ce diviseur de fréquence constitue une limite de temps pour distinguer entre deux positions différentes dans le temps du signal supplémentaire (3, 4, 4').

15. Système selon les revendications 14 et 4, caractérisé en ce que la sortie du diviseur de fréquence est reliée à l'entrée de données d'un registre à décalage (39) et en ce que l'apparation du signal supplémentaire (3, 4, 4') provoque l'application d'une impulsion à l'entrée d'horloge de ce registre à décalage.

16. Système selon les revendications 10 et 13, caractérisé en ce que la remise à l'état de départ est produite par un signal déclenché par le compteur (32) à une position de comptage préfixée.

17. Système selon une des revendications précédentes, caractérisé en ce que l'émetteur contient des moyens pour l'établissement temporaire d'une fréquence d'impulsions à des fins d'essais sur un stimulateur cardiaque artificiel programmable au moyen d'un champ magnétique extérieur et d'un interrupteur (13) à commande magnétique incorporé dans le stimulateur, la fréquence d'impulsions s'établissant effectivement étant caractéristique pour au moins un paramètre de fonctionnement, tel que la tension d'alimentation, et en ce que le récepteur contient des moyens, commandés par l'interrupteur à commande magnétique (13), qui font passer la fréquence d'impulsions du stimulateur à la fréquence d'impulsions pour essais, et maintiennent cette dernière fréquence tant que le champ magnétique extérieur est maintenu, avec préservation, en mémoire, du signal de commande correspondant au mode de fonctionnement du simulateur choisi et établi en dernier.

18. Système selon la revendication 17, caractérisé en ce que des moyens (13, 31, 51a, 52, 53) sont prévus qui, pendant la durée du maintien du champ magnétique, commandent au moins un élément de détermination de fréquence (résistances R 18 à R 25) et envoient au circuit de décodage (48) sur lequel agit le signal de commande numérique un signal amenant ce circuit de décodage (48) à établir temporairement la fréquence d'impulsions pour essais.

19. Système selon la revendication 18, caractérisé en ce que des moyens (51a, 52, 53, 48) sont prévus pour l'enclenchement temporaire de la fréquence d'impulsions pour essais sous l'effet du changement de la valeur d'au moins un élément (résistances R 18 à R 25) de détermination de fréquence et/ou sous l'effet de la mise en circuit d'un autre élément (résistance R 25) à sa place.

**Fig. 1**

**Fig. 2**

Fig. 3

Fig. 4

# Fig. 4 a

# Fig. 6

# Fig. 6 a

0 1 8 2

0 002 213

| Nummer des zu übertragenden Bits | 1 | 2 | 3 | 4 | 5 | 6 | 7 | 8 |
|---|---|---|---|---|---|---|---|---|
| codiertes Steuersignal | 1 | 0 | 1 | X = 1 | X = 0 | X = 1 | 0 | 1 |

START-Taste

Freigabesignal für Oszillator

Oszillatortakt, Sender

Flipflop 22 Ausgang Q

Flipflop 23 Ausgang Q

Schieberegister 16 Ausgang OUT

ausgesendetes Signal

Vorderflanken

Rückflanken

Oszillatortakt, Empfänger

Schieberegister 39 Ausgang Q 1

Ausgang Q 2

Ausgang Q 3

Ausgang Q 4

Ausgang Q 5

Ausgang Q 6

Ausgang Q 7

Ausgang Q 8

t

**Fig. 5**